# EUROPEAN PATENT APPLICATION

(11) **EP 2 322 207 A2**
(43) Date of publication of application: **18.05.2011**
(21) Application number: 09723300.1
(22) Date of filing: 17.03.2009
(51) Int. Cl.: A61K 38/42, C07K 14/805, A61P 7/00

(54) **OXYGEN-TRANSFERRING BLOOD SUBSTITUTE AND A PHARMACEUTICAL COMPOSITION (VARIANTS)**

(30) Priority: 18.03.2008 RU 2008109967
(71) Applicant: Nauchno Proizvodstennaya Kompaniya "MEDBIOFARM", Kaluzhskaya obl. 249031 (RU)
(72) Inventor: GONCHAROVA, Anna Yakovlevna, Kaluzhskaya obl. 249031 (RU); PODGORODNICHENKO, Vladimir Konstantinovich, Kaluzhskaya obl. 249032 (RU); ROZIEV, Rakhimdzhan Akhmetdzhanovich, Kaluzhskaya obl. 249031 (RU); HOMICHENOK, Viktor Vladimirovich, Kaluzhskaya obl. 249030 (RU); TSYB, Anatoliy Fedorovich, Kaluzhskaya obl. 249039 (RU); BRUSKOVA, Olga Borisovna, Kaluzhskaya obl. 249013 (RU)
(74) Representative: Reichert, Sabine
(86) International application number: PCT/RU2009/000129
(87) International publication number: WO 2009/116894

(57) **Abstract**

The invention relates to medicine, in particular to polyhemoglobin-based blood substitutes. The invention can be used for producing blood substituting solutions comparable in terms of a gas transportation efficiency (oxygen transfer) with human blood erythrocytes. The inventive oxygen-transferring blood substitute is based on the hemoglobin which is polymerised by glutaraldehyde produced from animal blood and differs in that it is in the form of a dry substance and contains not less than 90% of polymerised hemoglobin, the molecular mass of which ranges from 192 000 to 320 000 Da and the methemoglobin content in the blood substitute is equal to or less than 5%.

## Description

The present invention relates to medicine, namely blood substitutes based on polyhemoglobin. The invention can be used for the production of blood-substituting solutions comparable to the efficiency of gas transport (transport of oxygen) with erythrocytes of human blood.

A blood substitute / L. R. Sehgal, A. L. Rosen, S. A. Gould, H.L. Sehgal, G.S. Moss / Transfusion. - 1983. - V. 23. - N 2. - P. 158-162 /, of polymeric hemoglobin, is widely known.

The main drawback of the known blood substitute is its relatively high cost, which is due to the fact that the blood substitute for use expensive biologically active agent - pyridoxal-b-phosphate (PF). There is also a danger of incidence of free-PF in the final product, which can lead to undesirable consequences.

The blood substitute Gelenpol, /RU 2132687, A61 K35/18, A61 K38/42, 1999/, which is a mixture of hemoglobin tetramer and oligomers with various chain lengths, is also well-known. Oligomers are obtained by cross-linking the hemoglobin tetramers with glutaraldehyde and modified glutamic acid.

The disadvantage of Gelenpol is that packed red blood cells with a shelf life of no more than 36 days are required for its preparation, and this may adversely affect the stocks of donated blood.

The disadvantages also include the fact that the blood substitute has a large percentage of hemoglobin tetramer, which dissociates in the blood into dimers which have a nephrotoxic effect. The tetramer also causes vasoconstrictor effect by binding nitric oxide, and in addition, the tetramer is rapidly eliminated from the blood stream without being able to perform the gas-transport function.

The blood substitute according to patent RU 2203087, derived from donated blood, is also well-known. This blood substitute is an aqueous solution piridoxilated, polymerized hemoglobin, which contains approximately 16% hemoglobin polymer with a molecular mass of approximately 128, approximately 26% hemoglobin polymer with a molecular mass of approximately 192, and approximately 58% hemoglobin polymer with a molecular weight of about 256. This blood substitute is prepared using carbon monoxide, which increases the cost and lengthens the process; moreover, there is an admixture of the toxic carboxyhemoglobin (up to 1.5%) in the final product. The described technology allows for the use of blood of other mammals, as confirmed by the authors.

The hemoglobin-based blood substitute made by the biopure company, including hemoglobin from the red blood cells of cattle, Patents US 5,084,558 (1992) and US 6,506,725 (2003), is also well-known.

This blood substitute has the following parameters: distribution of the molecular weight of the polyhemoglobin is in the range of 68,000 - 500,000 daltons, more than 90%, with the polyhemoglobin content more than 50%, methemoglobin content of less than 20%, and endotoxin content of less than 0.02 units/ml.

The disadvantages of this blood substitute are high levels of tetramer and methemoglobin. The high tetramer content has a nephrotoxic effect and methemoglobin is toxic. In addition, the blood substitute is prepared using degassed solution, and therefore when introduced into the organism in conditions of hemorrhagic shock, can bind to dissolved oxygen in the blood, causing additional hypoxia. Biopure's product is a ready-to-use product with a fixed concentration of hemoglobin in a defined medium (modified lactated Ringer - Locke solution). In this regard, the possibility of changing the concentration of hemoglobin in a solution is limited and a change in solvent composition is not possible. Meanwhile, there may be situations where the use of this solvent is not desirable and a plasma substitute with different properties and different hemoglobin may turn out to be optimal.

The present invention is to create a blood substitute devoid of these shortcomings.

In order to solve this problem, a blood substitute with oxygen transport function and based on polymerized glutaraldehyde hemoglobin derived from animal blood is proposed. A distinctive feature of the proposed blood substitute is that the molecular weight distribution of 90% of the polyhemoglobin is in the range of 192,000 - 320,000 Da and the methemoglobin content is less than 5%. The blood substitute is proposed to be made in the form of a dry substance, such as a powder or granules, with a moisture content of no more than 7%.

The proposed blood substitute may also contain glucose and/or ascorbic acid.

The distribution of molecular weight of 90% of polyhemoglobin in the range of 192,000 - 320,000 Da and methemoglobin content less than 5% improve the quality of the blood substitute by eliminating unwanted side effects. It is known that polymerized hemoglobin has less cooperative effect than native hemoglobin, which reduces the Hill coefficient /Preparation and integral vitro characteristics of polymerized pyridoxylated hemoglobin, L.R. Sehgalet al., TRANSFUSION, Vol. 23, No. 2-1983, p. 158 - 162/. This effect is possibly due to steric hindrance to access of oxygen to the heme. The distribution of molecular weight in the specified range can bring gas transport properties of the claimed blood substitute closer to that of native hemoglobin due to the greater access of heme to oxygen.

Administration of glucose allows the use of freeze-drying to produce dry product, and ascorbic acid increases the shelf life of the preparation.

Making blood substitute in the form of dry substance increases its shelf life and strengthens its anti-shock effect. In addition, it expands the set of specific therapeutic effects through the ability to dissolve it in one of the accompanying plasma substitute solutions. The choice of solution is determined by the cause that led to the need to transfuse blood substitute, the condition of the patient, and the nature of the proposed therapy (treatment process).

Consequently, the technical result is achieved.

The blood substitute is prepared using osmotic hemolysis of the red blood cells of cattle. Therefore, the erythrocytic mass derived from the stabilized cow's blood by separation, is subjected to hemolysis with water for injection. Stroma (erythrocyte membrane) is separated by microfiltration using filters with pore diameters of 50, 20, 10, 5, and 1 microns. The filtrate is treated with *concentrated* sodium chloride solution for planting non-heme proteins. The solution is again filtered through filters with pore diameters of 5, 1, and 0.65 microns and subjected to the process of ultrafiltration with 300 kDa cut-off membranes, and sterilizing filtration is performed.

The concentration of native hemoglobin and of the products of its chemical modification is determined spectrophotometrically by using a cyanmethemoglobin derivative at a wavelength of 540 Nm /M.S. Kushakovsky / Clinical forms of damage to the hemoglobin, Leningrad: Medicine, 1968. - p. 23 /. Reagent grade sodium chloride ("chemically pure," GOST 42-2572-88), glucose (FS 42-2419-86), ascorbic acid (HF X, S - 6), and isotonic solution (GF Xl, First edition, p. 175) are used.

Polymerization. 1-10 wt.% aqueous solution of hemoglobin is deoxygenated, and at a temperature of 4°-8° C, 1 - 5 wt.% aqueous solution of glutaraldehyde is added at a molar ratio of *glutaraldehyde: hemoglobin* (10: 1) - (20: 1).

The reaction is completed by adding an aqueous solution of sodium borohydride *(pH 8-9).*

The polyhemoglobin solution is subjected to *ultrafiltration on cut-off membranes* of 450 kDa, and then for *concentration and diafiltration washing,* it is applied to the membranes filtering out polymers that are less than 150 kDa. The polyhemoglobin solution is washed with diafiltration to remove the tetramer and low molecular weight compounds, and then it is concentrated up to 10% polyhemoglobin and stored for drying. The quality of the preparation in terms of compliance with the fractional composition of polyhemoglobin is controlled through electrophoresis and *gel-permeation* chromatography.

Among the preparations with hemodynamic (anti-shock) effect, polyoxidin, containing 1.5% solution of polyethylene glycol, with a molecular mass of 20 kDa in 0.9% sodium chloride solution with the addition of potassium iodide, is proposed as a solvent for the claimed blood substitute. Polyoxidin has an anti-shock effect, as well as the ability to cause autohemodilution and retain fluid in the bloodstream. Polyoxidin restores capillary blood flow with its disaggregating effect.

Hemodez, a 6% solution of polyvinylpyrrolidone with a molecular mass of 12.6 kDa, with the addition of salts balanced by ionic composition, is proposed to be used as a detoxication preparation.

Ringer's solution, which contains ingredients in mmol/l Na- 147, K-4.0, Ca - 2.3, Cl - 155, and HCO₃ - 1.2, is recommended to be used as a regulator of water-salt metabolism and acid-base composition.

Mafusol - a solution of salts (sodium chloride, potassium chloride and magnesium chloride) and sodium fumarate - is recommended to be used as the polyfunctional (complex) preparation.

The recommended concentration of blood substitute in solutions is 1%.

Example 1. Stable bovine blood, chilled to +2°+4° C, in 10-liter plastic containers, total amount 30 liters, is passed from the refrigerating chamber through the gateway to the industrial premises. Blood is fed into the receptacle of operating separator Al-FMC with the aid of a peristaltic pump. Separation is carried out at 4000 rpm. The resulting erythromass in the amount of 12 liters enters the hemolysis container.

After introduction of 12 liters of erythromass in a disposable Flexel 3D bio-bag (E 1) for hemolysis with a volume of 50 liters (all tanks are equipped with cooling jacket), a peristaltic pump is switched on for mixing, and 36 liters of water is added for injection (BIA) with a temperature of +6 to +8° C (temperature controlled by the sensor in the palletank) from the collection tank, located in the solutions preparation chamber. Hemolysis takes place within 40 minutes while stirring.

The resulting hemolysate is pumped over cartridge filters with a pore size of 50, 20, 10, 5, and 1 microns, placed sequentially, and then into the Flexel bio-bag to precipitate non-heme proteins.

After filtration, the solution is fed into the bio-bag for the precipitation of non-heme proteins in the amount of 50 liters. After pumping the entire volume, concentrated

NaCl solution (33.3%) is fed into the container in an amount of 0.84 kg (BR.6). The precipitation lasts 30 minutes.

After the precipitation of non-heme proteins, the hemoglobin solution is pumped to the pump cartridge filters with a pore size of 5, 1, and 0.65 microns, arranged sequentially, and subsequently into a container for the concentration of hemoglobin solution.

Purification of hemoglobin solution from high-molecular-weight admixtures in is at the ultrafiltration plant with membranes, filtering out material with a molecular mass of over 300 kDa. Concentration of the hemoglobin solution takes place at the ultrafiltration plant with membranes, splitting out materials with molecular mass of more than 70 kDa, to a concentration of 10% hemoglobin. A sterilizing filtration is performed on the same setup with the use of membranes with a pore size of 0.2 microns.

After concentration, 23.5 liters of of the hemoglobin solution enters the gas-vortex reactor. A stream of sterile nitrogen is fed into the reactor (controlled by a rotameter), for intermixture and deoxygenation. Deoxygenation is carried out until oxygen concentration in the equilibrium gas phase of 1.0-2.0 vol. % is achieved (in the reactor is located the oxygen concentration sensor).

A solution of 2.5% glutaraldehyde in an amount of 1.83 kg is pumped to the reactor R1, which contains deoxyhemoglobin (TP.3.1.), for 40 min. The reaction is carried out with stirring for 1 hour at a temperature of +6° to +8° C in a nitrogen flow.

The reaction is stopped using a solution of sodium borohydride added in the amount of 0.2 kg into the reactor with the polyhemoglobin solution. The reaction occurs for 30 minutes while stirring. Then the reaction mixture is pressure-pumped with nitrogen (excess pressure 0.5 - 1 atm) in the diafiltration container.

The reaction mixture is pressure-pumped with nitrogen (pressurized 0.5 - 1 atm.) from the bioreactor and is introduced into a 50-liter bio-bag. Thereafter, the polyhemoglobin solution is pumped via a replenishment pump for 300kDa diafiltration to remove the high-molecular weight compounds (of more than 6 molecules of hemoglobin). After diafiltration, 20 liters of the solution is fed to the replenishment pump for concentration and washing diafiltration at 1 00kDa. The solution is washed with 150 liters of water for injection, for removal of the modified hemoglobin (intramolecular bonded tetramer) and low molecular weight compounds, and concentrated to 20 liters.

The wash solution is directed to wastewater treatment plants.

Sterile 40% glucose solution in the amount of 4.83 liters and 0.2 liters of 13,6% sterile solution of ascorbic acid is added into the bio-bag with the polyhemoglobin solution added through the sterile connection connector, and the solution is stirred. Then the prepared solution is fed through a peristaltic pump to the sterilizing filtration installation with a pore size of 0.22 microns. Filtrate enters the holding tank for delivery for drying.

Lyophilic or vacuum drying providing for the production of sterile powder in an amount of 4.8 kg is used for drying the preparation.

Comparable values of the Hill coefficient are listed in the following table:1.

**Table 1**

| Hill coefficient | native hemoglobin | Biopure blood substitute | The claimed blood substitute |
|---|---|---|---|
| | 2.0 | 1.4 | 1.8 |

Treatment efficiency of the resulting blood substitute was evaluated on a model of hemorrhagic shock in dogs. Sterile freeze-dried polyhemoglobin containing glucose and ascorbic acid was used for the study. The powder was diluted with saline solution. Ten mongrel dogs - males weighing 10-20 kg - were used for the experiment, which artificially induced hemorrhagic shock in acute blood loss with prolonged hypotension. Estimated blood loss was 45 - 55 ml/kg, and the duration of hypotension at the level of blood pressure equal to 40 mm Hg. was 60 - 70 min. Intravenous solution of polymerized glutaraldehyde-modified hemoglobin was introduced during the pronounced disorders of the systemic hemodynamics, oxygen transport and acid-base state of the body, caused by massive blood loss and prolonged hypotension. The preparation obtained through the claimed method was introduced iat the same volume as the blood loss. Ten out of ten of the dogs in the study survived, wherein it was revealed that the infusion of the solution with a low concentration of polyhemoglobin (0.8g/dl) provides not only a persistent restoration of systemic hemodynamics, but also a higher level of tissue oxygen consumption.

When a preparation with the characteristics of the prototype in a similar experiment was used, nine out of the ten animals survived.

## Claims

1. A blood substitute with oxygen transport function, based on glutaraldehyde-polymerized hemoglobin derived from animal blood, distinguished by the fact that it is a dry substance and contains at least 90% polymerized hemoglobin with molecular weight in the range of 192,000 - 320,000 Da and the methemoglobin content in the blood substitute no more than 5%.

2. The blood substitute according to Claim 1, distinguished by the fact that it additionally contains glucose.

3. The blood substitute of Claim 1, distinguished by the fact that it additionally contains ascorbic acid.

4. The blood substitute according to Claim 1, distinguished by the fact that is a powder with a humidity no greater than 7%.

5. The blood substitute according to Claim 4, wherein the granules have a moisture content of no greater than 7%.

6. A pharmaceutical composition containing the blood substitute according to Claim 1 and polioxydin containing a 1.5% solution of polyethylene glycol with a molecular mass of 20 kDa in a 0.9% sodium chloride solution with the addition of potassium iodide.

7. A pharmaceutical composition containing the blood substitute according to Claim 1, and a 6% solution of polyvinylpyrrolidone with a molecular mass of 12.6 kDa, with the addition of salts balanced in terms of ionic composition.

8. A pharmaceutical composition containing the blood substitute according to Claim 1 and Ringer's solution.

9. A pharmaceutical composition according to Claim 8, distinguished by the fact that the Ringer's solution contains Na -147, K - 4.0, Ca - 2.3, Cl - 155, and HCO₃-1.2 mmol/l.

10. A pharmaceutical composition containing the blood substitute according to Claim 1 and a solution of sodium chloride, potassium chloride and magnesium chloride and sodium fumarate.
